# EUROPEAN PATENT APPLICATION

(11) **EP 3 545 838 A1**
(43) Date of publication of application: **02.10.2019**
(21) Application number: 18164239.8
(22) Date of filing: 27.03.2018
(51) Int. Cl.: A61B 5/11

(54) **PROVIDING AN OUTPUT INDICATIVE OF A LEFT VENTRICULAR EJECTION TIME**

(71) Applicant: Nokia Technologies Oy, 02610 Espoo (FI)
(72) Inventor: RAJALA, Satu, 36200 Kangasala (FI); OKSALA, Niku, 33700 Tampere (FI); LINDHOLM, Harri, 00320 Helsinki (FI)
(74) Representative: Whiting, Gary

(57) **Abstract**

An apparatus and method is described comprising: obtaining a ballistocardiographic signal for a subject; determining an I-J interval from the ballistocardiographic signal, including detecting peaks and troughs in the ballistocardiographic signal; and providing an output indicative of a left ventricular ejection time based on the determined I-J interval.

## Description

### Field

The present specification relates to providing an output indicative of a left ventricular ejection time.

### Background

A heart is a muscular organ that pumps blood through blood vessels. The human heart includes four chambers: namely a right atrium, a left atrium, a right ventricle and a left ventricle. The left atrium receives oxygenated blood from the lungs and the left ventricle pumps the oxygenated blood, via the aorta, around the body. Left ventricular ejection time (LVET) is the time difference between the opening and closing of the aortic valve of the heart. LVET is an important part of the pumping functionality of the heart and has a role in the efficient ejection of blood from the heart.

There is a need for alternative arrangements for providing an output indicative of left ventricular ejection time.

### Summary

In a first aspect, this specification describes an apparatus comprising: means for obtaining (e.g. receiving) a ballistocardiographic signal for a subject; means for determining an I-J interval from the ballistocardiographic signal, including detecting peaks and troughs in the ballistocardiographic signal; and means for providing an output indicative of a left ventricular ejection time based on the determined I-J interval.

The output indicative of the left ventricular ejection time may provide one or more of: an estimate of the left ventricular ejection time; an estimate of a change in the left ventricular ejection time; and an estimate of a rate of change in the left ventricular ejection time.

The detecting of peaks and troughs in the ballistocardiographic signal may comprise detecting I and J waves in the ballistocardiographic signal.

The means for obtaining the ballistocardiographic signal may comprise determining the ballistocardiographic signal from a measured force signal of the subject.

The means for obtaining the ballistocardiographic signal for the subject may comprise means for receiving a signal from a force sensor, the force sensor suitable for measuring a force from the subject. The means for obtaining the ballistocardiographic signal for the subject may further comprise means for determining the ballistocardiographic signal by converting the force signal into the ballistocardiographic signal.

The apparatus may further comprise means for determining a rate of change of the estimated left ventricular ejection time.

The means may comprise: at least one processor; and at least one memory including computer program code, the at least one memory and computer program code configured to, with the at least one processor, cause the performance of the apparatus.

In a second aspect, this specification describes a method comprising: obtaining a ballistocardiographic signal for a subject; determining an I-J interval from the ballistocardiographic signal, including detecting peaks and troughs in the ballistocardiographic signal; and providing an output indicative of a left ventricular ejection time based on the determined I-J interval.

The determined I-J interval maybe provided as an estimated left ventricular ejection time.

Detecting peaks and troughs in the ballistocardiographic signal may comprise detecting I and J waves in the ballistocardiographic signal.

Obtaining ballistocardiographic data may comprise determining ballistocardiographic data by converting obtained force data into ballistocardiographic data.

The method may further comprise determining the obtained force data through measurement.

The method may further comprise identifying changes in the estimated left ventricular ejection time over time, such as identifying trends in the changes in the estimated left ventricular ejection time. This maybe useful, for example, in combination with other basic cardiac parameters, for example, for obtaining useful profiles. As an example, among healthy subjects, the left ventricular ejection time (LVET) and heart rate are inversely correlated and the absence of this relationship may reflect cardiac dysfunction. The assessment of the temporal changes may be useful in long-term follow-up of heart health or in the evaluation of heart health during acute challenges.

In a third aspect, this specification describes an apparatus configured to perform any method as described with reference to the second aspect.

In a fourth aspect, this specification describes computer-readable instructions which, when executed by computing apparatus, cause the computing apparatus to perform any method as described with reference to the second aspect.

In a fifth aspect, this specification describes a computer readable medium comprising program instructions stored thereon for performing at least the following: obtaining a ballistocardiographic signal for a subject; determining an I-J interval from the ballistocardiographic signal, including detecting peaks and troughs in the ballistocardiographic signal; and providing an output indicative of a left ventricular ejection time based on the determined I-J interval.

In a sixth aspect, this specification describes a non-transitory computer-readable medium comprising program instructions stored therefore for performing at least the following: obtaining a ballistocardiographic signal for a subject; determining an I-J interval from the ballistocardiographic signal, including detecting peaks and troughs in the ballistocardiographic signal; and providing an output indicative of a left ventricular ejection time based on the determined I-J interval.

In a seventh aspect, this specification describes an apparatus comprising: at least one processor; and at least one memory including computer program code which, when executed by the at least one processor, causes the apparatus to: obtain a ballistocardiographic signal for a subject; determine an I-J interval from the ballistocardiographic signal, including detecting peaks and troughs in the ballistocardiographic signal; and provide an output indicative of a left ventricular ejection time based on the determined I-J interval.

### Brief description of the drawings

Example embodiments will now be described, by way of non-limiting examples, with reference to the following schematic drawings, in which:
FIG. 1 is a plot showing an example of idealised ballistocardiogram (BCG) data;
FIG. 2 is a highly schematic block diagram of a system in accordance with an example embodiment;
FIG. 3 is a flow chart showing an algorithm in accordance with an example embodiment;
FIG. 4 shows a chair incorporating a force sensor in accordance with an example embodiment;
FIG. 5 is a flow chart showing an algorithm in accordance with an example embodiment;
FIG. 6 is a flow chart showing an algorithm in accordance with an example embodiment;
FIG. 7 is a block diagram of a system in accordance with an example embodiment; and
FIGS. 8a and 8b show tangible media, respectively a removable memory unit and a compact disc (CD) storing computer-readable code which when run by a computer perform operations according to example embodiments.

### Detailed description

Systolic time intervals are related to the mechanical function of the heart. Two parameters that are sometimes measured are: pre-ejection period (PEP) and left ventricular ejection time (LVET).

The pre-ejection period (PEP) maybe defined as the time difference between the beginning of electrical activation of the heart (Q-wave in electrocardiogram (ECG) data) and the beginning of blood ejection to the aorta (aortic valve opening). Left ventricular ejection time (LVET) may be defined as the time difference between the aortic valve opening and the aortic valve closing.

Ballistocardiogram (BCG) is a measurement of mechanical activity of the heart. A sudden motion of blood mass in one direction produces recoil of the body in the opposite direction. Ballistocardiogram (BCG) data is based on the measurement of this recoil.

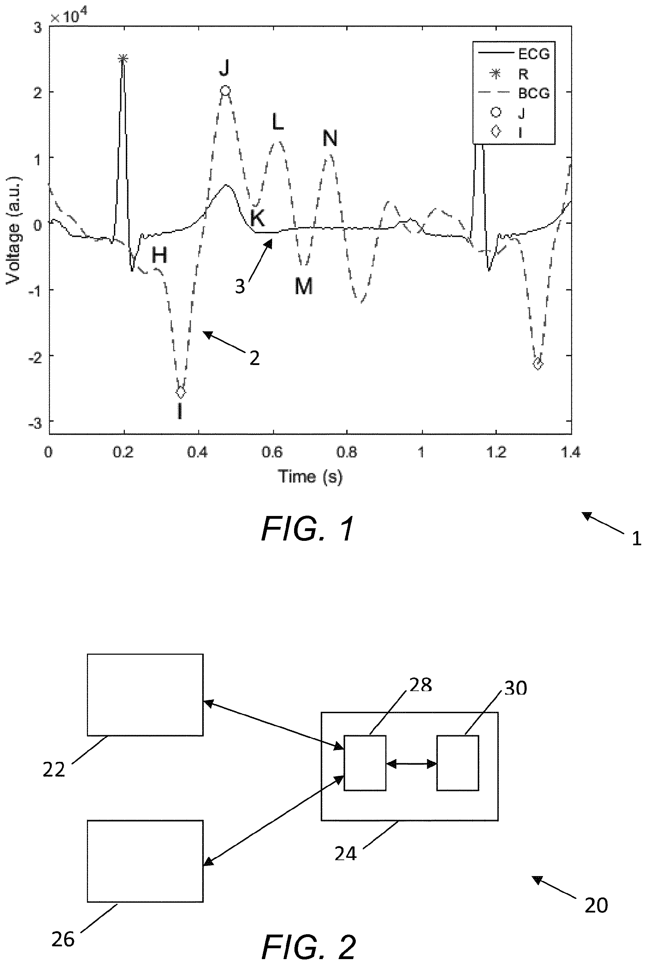

FIG. 1 is a plot, indicated generally by the reference numeral 1, showing an example of idealised ballistocardiogram (BCG) data (indicated generally by the reference numeral 2) and idealised electrocardiogram (ECG) data (indicated generally by the reference numeral 3). The BCG data 2 consists of several separate waves that collectively form an H-N wave. The plot 1 has units of voltage on the vertical axis. It should be noted that the raw signals for the BCG data 2 and the ECG data 3 may have different magnitudes and therefore one of them may be scaled to make them easier to compare. In the example measurements shown in FIG. 1, the BCG signal was scaled using a voltage divider.

FIG. 2 is a schematic block diagram of a system, indicated generally by the reference numeral 20, in accordance with an example embodiment. The system 20 comprises a BCG sensor 22 (such as a force sensor, as described further below), a control module 24 and an output module 26. The control module 24 includes a processor 28 and a memory 30. The memory 30 may include computer program code which may be executed by the processor 28.

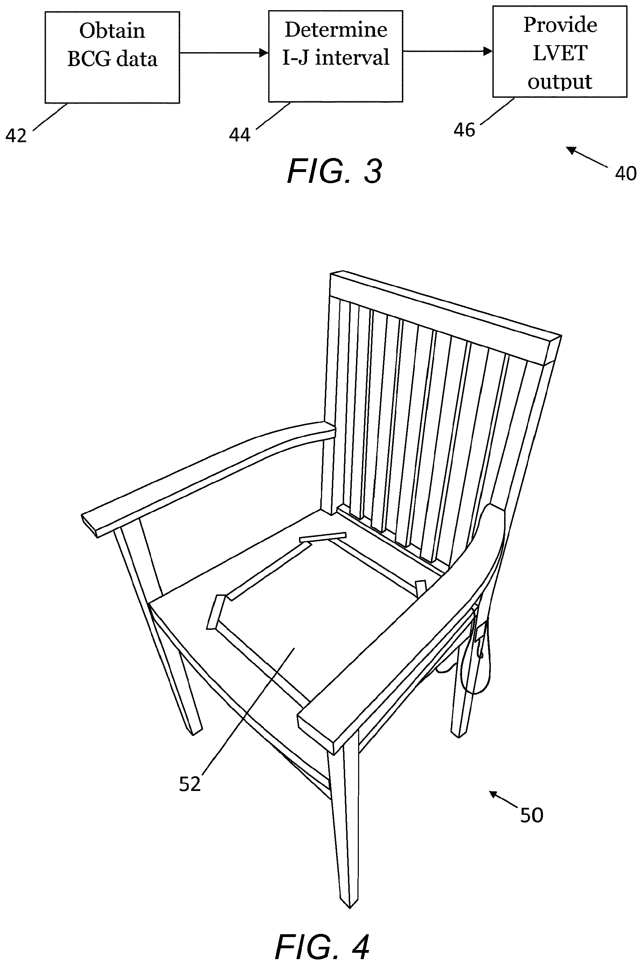

FIG. 3 is a flow chart showing an algorithm, indicated generally by the reference numeral 40, in accordance with an example embodiment. The algorithm 40 starts at operation 42 where BCG data is obtained, for example from the BCG sensor 22. The BCG data is provided, for example, to the processor 28.

At operation 44, an I-J interval in the BCG data is measured. As described further below, the I-J interval may be measured (for example by the processor 28) by identifying peaks and troughs in the BCG data and thereby identifying the I and J waves. The I-J interval is one option for providing an output indicative of the left ventricular ejection time.

Finally, at operation 46, LVET data is output using the output module 26. The LVET data may take a number of different forms. The LVET data may be an estimate of an absolute data (for example, based on a measurement of the I-J interval), but this is not essential to all embodiments. For example, the LVET data provided by the output module 26 may provide an estimate of a change in LVET data, or a rate of change of LVET data.

By way of example, if a reference echocardiogram (ECG) is measured, then a calibration coefficient may be determined and used to convert the measured I-J intervals (operation 44 above) to actual LVET values (for output in operation 46 above).

As discussed above, BCG data may be determined in operation 42 by measuring a recoil force produced in reaction to a sudden motion of blood mass caused by heart contractions during the cardiac cycle. The recoil force is typically in a longitudinal direction along an axis parallel to the spine. BCG data may, for example, be measured with force or acceleration sensors, with a user in a standing, sitting or supine position.

FIG. 4 shows a chair, indicated generally by the reference numeral 50, incorporating a force sensor 52 in accordance with an example embodiment. The force sensor 52 can be used to detect variations in force that are related to heart activity, thereby generating the BCG data. The force sensor 52 may, for example, be a ferroelectret sensor (e.g. a thin film of polymer foams exhibiting piezoelectric properties). The use of a ferroelectret sensor is not essential to all embodiments. For example, the functionality of the sensor 52 may be implemented using strain gauge sensors, force plates or piezoelectric film materials. Moreover, the use of the chair 50 is not essential to all embodiments. For example, such data could be obtained from a force sensor incorporated into a bed on which a subject is lying or into a weighing scale on which the subject is standing.

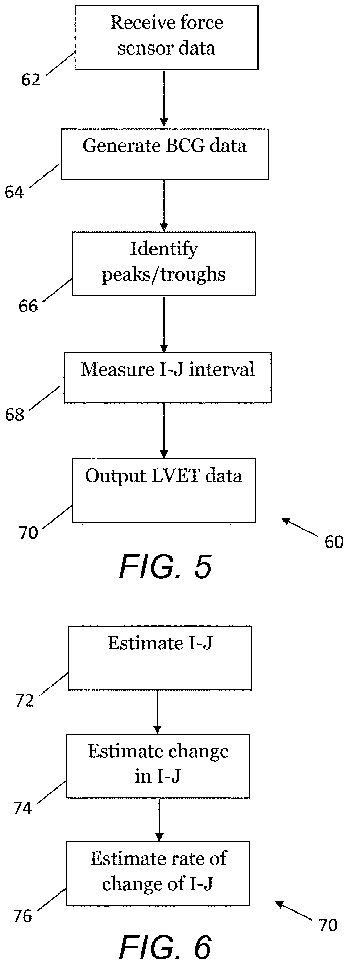

FIG. 5 is a flow chart showing an algorithm, indicated generally by the reference numeral 60, in accordance with an example embodiment.

The algorithm 60 starts at operation 62 where force sensor data is obtained. The force sensor data may be received from the force sensor 52 described above. At operation 64, BCG data is generated on the basis of the force data obtained in operation 62. It should be noted that operations 62 and 64 of the algorithm 60 provide an example implementation of the operation 42 described above, but are not essential to all embodiments. For example, BCG data may be obtained in other ways. In one form of the invention, the force data is used as the BCG data without further conversion (other than, for example, a scaling step, as described above with reference to FIG. 1). Accordingly, in some implementations, the operation 64 maybe omitted.

At operation 66, peaks and troughs in the BCG data are identified. As can be seen from the example BCG data shown in FIG. 1, identifying peaks and troughs enables the I-wave and J-wave data to be identified.

At operation 68, the I-J interval (ms) is measured from the peaks identified in BCG signal of the same cardiac cycle.

Finally, at operation 70, LVET data is generated and output (for example using the output module 26). The LVET data could take different forms. For example, the LVET data could be an estimate of the absolute value of the left ventricular ejection time (based, for example, on the measured I-J interval). Alternatively, the LVET data output in operation 70 could be an indication of a change in LVET or a rate of change of LVET.

FIG. 6 is a flow chart showing an algorithm, indicated generally by the reference numeral 70, in accordance with an example embodiment.

The algorithm 70 starts at operation 72, where an I-J interval is determined (e.g. through measurement as implemented, for example, in the operation 68 described above). At operation 74, a change in the I-J data is estimated. The operation 74 may be implemented, for example, by comparing a current I-J interval with a previous I-J interval, for example stored in the memory 30 of the control module 28 described above. At operation 76, a rate of change of the I-J interval may be estimated. The rate of change may be estimated on the basis of the change in I-J estimated in operation 74 divided by the time between two successive measurements.

The algorithm 70 is provided by way of example only; many alternatives are possible. For example, it is not essential to all embodiments to generate the I-J interval, the change in the I-J interval and the rate of change of the I-J interval. Indeed, one or more of those estimates may be omitted. Moreover, many methods for generating the relevant outputs will be apparent to those skilled in the art. For example, a weighted average may be used for estimating the rate of change of the I-J interval, rather than simply the difference between two measurement points.

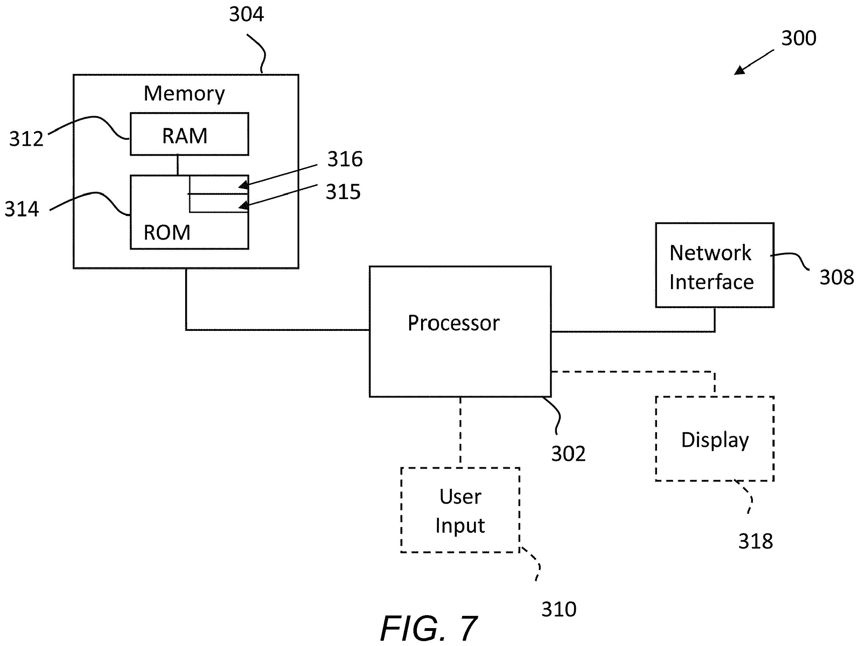

For completeness, FIG. 7 is a schematic diagram of components of one or more of the modules described previously (e.g. the control module 24), which hereafter are referred to generically as processing systems 300. A processing system 300 may have a processor 302, a memory 304 closely coupled to the processor and comprised of a RAM 312 and ROM 314, and, optionally, user interface 310 and a display 318. The processing system 300 may comprise one or more network interfaces 308 for connection to a network, e.g. a modem which may be wired or wireless.

The processor 302 is connected to each of the other components in order to control operation thereof.

The memory 304 may comprise a non-volatile memory, a hard disk drive (HDD) or a solid state drive (SSD). The ROM 314 of the memory 304 stores, amongst other things, an operating system 315 and may store software applications 316. The RAM 312 of the memory 304 is used by the processor 302 for the temporary storage of data. The operating system 315 may contain code which, when executed by the processor, implements aspects of the algorithms 40, 60 or 70.

The processor 302 may take any suitable form. For instance, it may be a microcontroller, plural microcontrollers, a processor, or plural processors.

The processing system 300 may be a standalone computer, a server, a console, or a network thereof.

In some embodiments, the processing system 300 may also be associated with external software applications. These may be applications stored on a remote server device and may run partly or exclusively on the remote server device. These applications may be termed cloud-hosted applications. The processing system 300 may be in communication with the remote server device in order to utilize the software application stored there.

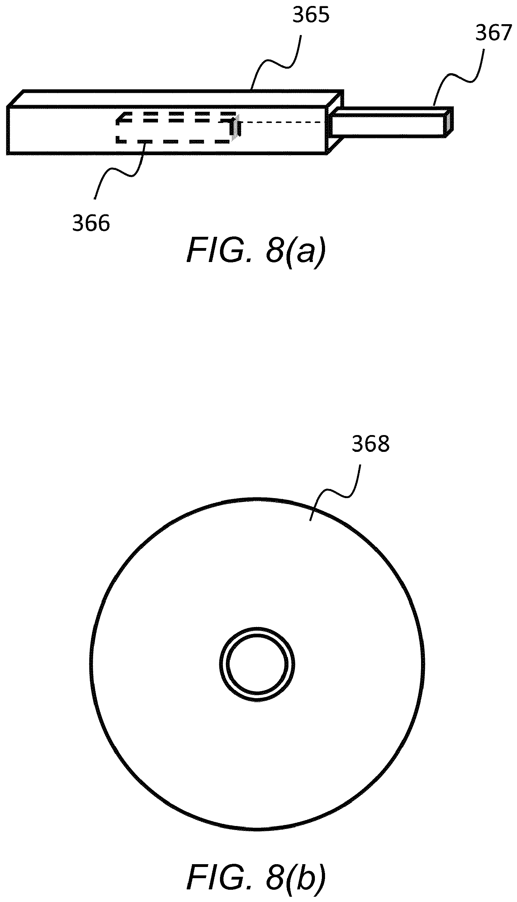

FIG. 8a and FIG. 8b show tangible media, respectively a removable memory unit 365 and a compact disc (CD) 368, storing computer-readable code which when run by a computer may perform methods according to embodiments described above. The removable memory unit 365 may be a memory stick, e.g. a USB memory stick, having internal memory 366 storing the computer-readable code. The memory 366 may be accessed by a computer system via a connector 367. The CD 368 may be a CD-ROM or a DVD or similar. Other forms of tangible storage media may be used.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices and other devices. References to computer program, instructions, code etc. should be understood to express software for a programmable processor firmware such as the programmable content of a hardware device as instructions for a processor or configured or configuration settings for a fixed function device, gate array, programmable logic device, etc.

As used in this application, the term "circuitry" refers to all of the following: (a) hardware-only circuit implementations (such as implementations in only analogue and/or digital circuitry) and (b) to combinations of circuits and software (and/or firmware), such as (as applicable): (i) to a combination of processor(s) or (ii) to portions of processor(s)/ software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present.

If desired, the different functions discussed herein maybe performed in a different order and/or concurrently with each other. Furthermore, if desired, one or more of the above-described functions may be optional or may be combined. Similarly, it will also be appreciated that the flow diagrams of FIGS. 3, 5 and 6 are examples only and that various operations depicted therein maybe omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the above describes various examples, these descriptions should not be viewed in a limiting sense. Rather, there are several variations and modifications which may be made without departing from the scope of the present invention as defined in the appended claims.

## Claims

1. An apparatus comprising:
means for obtaining a ballistocardiographic signal for a subject;
means for determining an I-J interval from the ballistocardiographic signal, including detecting peaks and troughs in the ballistocardiographic signal; and
means for providing an output indicative of a left ventricular ejection time based on the determined I-J interval.

2. An apparatus as claimed in claim 1, wherein the output indicative of the left ventricular ejection time provides one or more of: an estimate of the left ventricular ejection time; an estimate of a change in the left ventricular ejection time; and an estimate of a rate of change in the left ventricular ejection time.

3. An apparatus as claimed in claim 1 or claim 2, wherein the detecting of peaks and troughs in the ballistocardiographic signal comprises detecting I and J waves in the ballistocardiographic signal.

4. An apparatus as claimed in any one of claims 1 to 3, wherein the means for obtaining the ballistocardiographic signal comprises determining the ballistocardiographic signal from a measured force signal of the subject.

5. An apparatus as claimed in any one of the preceding claims, wherein the means for obtaining the ballistocardiographic signal for the subject comprises:
means for receiving a signal from a force sensor, the force sensor suitable for measuring a force from the subject.

6. An apparatus as claimed in any one of the preceding claims, further comprising means for determining a rate of change of the estimated left ventricular ejection time.

7. An apparatus as claimed in any one of the preceding claims, wherein the means comprise:
at least one processor; and
at least one memory including computer program code, the at least one memory and computer program code configured to, with the at least one processor, cause the performance of the apparatus.

8. A method comprising:
obtaining a ballistocardiographic signal for a subject;
determining an I-J interval from the ballistocardiographic signal, including detecting peaks and troughs in the ballistocardiographic signal; and
providing an output indicative of a left ventricular ejection time based on the determined I-J interval.

9. A method as claimed in claim 8, wherein the determined I-J interval is provided as an estimated left ventricular ejection time.

10. A method as claimed in claim 8 or claim 9, wherein detecting peaks and troughs in the ballistocardiographic signal comprises detecting I and J waves in the ballistocardiographic signal.

11. A method as claimed in anyone of claims 8 to 10, wherein obtaining ballistocardiographic data comprises determining ballistocardiographic data by converting obtained force data into ballistocardiographic data.

12. A method as claimed in claim 11, further comprising determining the obtained force data through measurement.

13. A method as claimed in any one of claims 8 to 12, further comprising identifying changes in the estimated left ventricular ejection time over time.

14. A method as claimed in claim 13, further comprising identifying trends in the changes in the estimated left ventricular ejection time.

15. A computer readable medium comprising program instructions stored thereon for performing at least the following:
obtaining a ballistocardiographic signal for a subject;
determining an I-J interval from the ballistocardiographic signal, including detecting peaks and troughs in the ballistocardiographic signal; and
providing an output indicative of a left ventricular ejection time based on the determined I-J interval.
